# EUROPEAN PATENT SPECIFICATION

(11) **EP 1 954 661 B1**
(45) Date of publication and mention of the grant of the patent: **19.10.2016**
(21) Application number: 06827435.6
(22) Date of filing: 03.11.2006
(51) Int. Cl.: C07C 17/23, C07C 17/25, C07C 17/278, C07C 21/18, C07C 19/10

(54) **METHOD FOR PRODUCING 2,3,3,3-TETRAFLUORO-1-PROPENE**
VERFAHREN ZUR HERSTELLUNG VON 2,3,3,3-TETRAFLUOR-1-PROPEN
PROCÉDÉ DE PRODUCTION DE 2,3,3,3-TÉTRAFLUORO-1-PROPÈNE

(30) Priority: 03.11.2005 US 733378 P
(43) Date of publication of application: 13.08.2008
(60) Divisional application: 11156860.6
(73) Proprietor: Honeywell International Inc., Morris Plains, NJ 07950 (US)
(72) Inventor: MUKHOPADHYAY, Sudip, Williamsville, NY 14221 (US); DUBEY, Rajesh, Buffalo, NY 14210 (US); SINGH, Rajiv R., Getzville, NY 14068 (US); SHIA, George Honeywell International Inc,, Morristown, NJ 07962-2245 (US)
(74) Representative: Cornish, Kristina Victoria Joy
(86) International application number: PCT/US2006/042936
(87) International publication number: WO 2007/056128

(56) References cited:
- WO-A-90/08748
- WO-A-98/42645
- WO-A-2005/042451
- US-A- 5 986 151
- US-A1- 3 505 417
- US-A1- 2006 106 263
- US-A1- 2006 217 577
- HASZELDINE, R. N. ET AL: "Addition of free radicals to unsaturated systems. XIII. Direction of radical addition to chloro-1,1-difluoroethylene" JOURNAL OF THE CHEMICAL SOCIETY, 1957, pages 2193-2197, XP009081235
- V. MONTANARI: "A Novel Synthesis of Perhalogenated Alkenes" J. ORG. CHEM., vol. 57, 1992, pages 5018-5019, XP002426455
- R. S. DICKSON; G. D. SUTCLIFFE: "Fluorocarbon-Aluminium Compounds" AUST. J. CHEM., vol. 25, 1972, pages 761-768, XP009081192
- MCDONIEL, J. BRIDGET ET AL: "Threshold Energy and Unimolecular Rate Constant for Elimination of HF from Chemically Activated CF3CF2CH3: Effect of the CF3 Substituent on the .alpha.-Carbon" JOURNAL OF PHYSICAL CHEMISTRY A, vol. 101, no. 7, 1997, pages 1334-1337, XP002426456

## Description

### BACKGROUND OF INVENTION

### (1) Field of Invention:

This invention relates to methods for the preparation of 2,3,3,3-tetrafluoro-1-propene (HFO-1234yf).

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze)) (HFO is hydrofluorolefin) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkanes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts, including a sizeable amount of carbon black. The carbon black is not only unwanted, it tends to deactivate the catalyst used in the process.

The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Catalyzed hydrogen reduction reactions have been disclosed for the preparation of fluorinated C3 hydrocarbons in U.S. Patent No. 5,545,777. The patent describes the reaction as being one in which a compound of formula (1)

C₃HₐCl_{b}F_{c} (1)

is converted by catalyzed hydrogen reduction to a compound of formula (2)

C₃Hₐ₊ₓCl_{b-y}F_{c-z} (2)

where a, b, c, x, y and z are integers satisfying the following conditions:
a≥0, b≥1, c≥2, x≥1, y ≥1, z ≥0, a+b+c=8, x=y+z, b-y≥0, and c-z≥2. Since the reactions disclosed in this patent require a reaction product in which a+b+c=8 and that x=y+z, it is not possible for the disclosed reaction product to include C3 olefins, which as mentioned above have been found to be desirable for use in many important applications.

Haszeldine, R.N. et al., J. Chem. Soc. (1957), 2193-2197 describes the addition of free radicals to unsaturated systems. In particular, it discusses the effect of free radical attack on chloro-1:1-difluoroethylene.

Montanari, V. et al., J. Org. Chem., 57, (1992), 5018-5019 discloses synthesising per halogenated alkenes.

Dickson, R.S. et al., Aust. J. Chem., 25, 1972, 761-768 relates to reactions of heptafluoro-3-iodepropane, 1,1,1,2,2-pentafluoride-3-iodepropane and heptafluoro-2-iodepropane with lithium tetrahydro aluminate.

McDoniel, J.B. et al., Journal of Physical Chemistry A, 101 (7), 1997, 1334-1337 provides a determination of the threshold energy and unimolecular rate constant for the elimination of HF from chemically activated CF₃CF₂CF₃.

WO 98/42645 relates to a process for reacting a saturated compound of the formula RF with an olefin of the formula R¹R²C=CR¹R² in the liquid phase in the presence of antimony pentafluoride catalyst.

Notwithstanding prior teachings applicants appreciate a continuing need for methods of efficiently preparing certain hydrofluorocarbons, particularly tetrafluorpropenes such as HFO-1234yf.

### SUMMARY OF THE INVENTION

Applicants have discovered a method for producing fluorinated organic compounds, including hydrofluoropropenes, which comprises converting at least one compound of formula (I):

CF₃CF₂CH₂Cl or CF₃CF=CHCl (I)

to the compound of formula (II)

CF₃CF=CH₂ (II).

The preferred converting step of the present invention comprises catalytic reduction of the compound of formula (I). The catalytic reduction step comprises in preferred embodiments introducing said compound of formula (I) to a reaction system under conditions effective to convert, and preferably convert at least about 50%, more preferably at least about 70%, and even more preferably at least about 90%, of said compound of formula (I), It is also generally preferred that said converting step produces a reaction product having at least about 20% selectivity, more preferably at least about 40% selectivity and even more preferably at least about 70% selectivity, to compound of formula (II), which is HFO-1234yf.

In certain preferred embodiments, the converting step comprises reacting a compound of formula (I) in the gas phase, in the liquid phase, or a combination of these, with gas phase reactions preferably occurring in the presence of catalyst.

### DETAILED DESCRIPTION OF PREFERRED EMBODIMENTS

One beneficial aspect of the present invention is that it enables the production of desirable fluroolefins using relatively high conversion and high selectivity reactions. In addition, the methods of the present invention provided reactions with relatively high yield and which are capable of obtaining relatively long catalyst life.

Furthermore, the present methods in certain preferred embodiments permit the products of the desirable fluoroolefins from relatively attractive starting materials. Ethylene and is halogentated derivates, such as tertrafluorethylene, may in certain embodiments be an advantageous starting material because such products are relatively easy to handle, and are generally readily available in commercial quantities and/or can be easily produced from other readily available materials. For example, the compounds of formula (I) can be synthesized by the catalyzed gas phase addition of CH₂FCl and CF₂=CF₂.

Thus, in certain embodiments the present methods include the step of reacting fluorinated C2 olefin, such as tetrafluoroethylene, with a C1 addition agent under conditions effective to produce a compound of formula (I)

CF₃CF₂CH₂Cl or CF₃CF=CHCl (I)

The fluorinated olefin reactant is a compound of formula (III)

CF₂=CY₂ (III)

selected from tetrafluoroethylene or chlorotrifluoroethylene, and the C1 addition agent comprises a compound of formula (IV) CH₂FCl (IV).

The reaction by which the compound of formula (III) is converted to a compound of formula (I) is sometimes referred to herein as an addition reaction.
formed by a process comprising a catalyzed C1 addition reaction, is then exposed to reaction conditions effective to produce a reaction product of formula (II). In one preferred aspect of the present invention, the conversion step comprises a reaction that is sometimes referred to herein as a reduction reaction and in other aspects as a dehydrohalogenation reaction. Preferred aspects of each of the preferred steps is described below.

### I. ADDITION REACTION

The reactant compound of formula (III) is
CF₂=CF₂ (sometimes referred to herein as "TFE") or CF₂=CFCl (sometimes referred to herein as "CTFE"). The fluorinated ethylene
compounds are reacted with CH₂FCl.

In certain preferred embodiments, the addition step comprises contacting, (preferably by introducing into a reactor) the compounds in an CH₂FCl: formula III mole ratio of from 1:1 to 200:1, more preferably from 1:1 to about 100:1 and even more preferably of from 2:1to 3:1. In preferred embodiments, comprising CH₂FCl and the
formula III compound, CF₂=CF₂, the CH₂FCl:TFE mole ratio of the feeds to the reactor are from 1:1 to 200:1, more preferably from 1:1 to 100:1 and even more preferably from 1.5:1 to 2:1.

It is contemplated that this reaction step can be carried out in the liquid phase or in the gas phase, or a combination of liquid/gas phases, and it is further contemplated that the reaction can be carried out batch wise, continuous, or a combination of these.

Thus, it is contemplated that the addition step, may be preformed using a wide variety of process parameters and process conditions in view of the overall teachings contained herein. However, it is preferred in certain embodiments that this reaction step comprise a gas phase reaction, preferably in the presence of catalyst, supported on carbon or unsupported, preferably a metal-based catalyst, such as antimony-based catalysts (such as SbF₃, SbF₅, and partially flourinated SbCl₃ or SbCl₅) aluminum-based catalyst (such as AlCl₃), iron-based catalyst such FeCl₃ including such catalysts on a carbon or other appropriate support. It is expected that many other catalysts may be used depending on the requirements of particular embodiments, and of course, two or more any of these catalysts, or other catalysts not named here, may be used in combination.

The gas phase addition reaction may be conducted, for example, by introducing a gaseous form of a compound of formula (III) and formula (IV) into a suitable reaction vessel or reactor. Preferably the vessel is comprised of materials which are resistant to corrosion, such as Hastelloy, Inconel, Monel and/or fluoropolymers linings. Preferably the vessel contains catalyst, for example a fixed or fluid catalyst bed, packed with a suitable addition catalyst, with suitable means to heat the reaction mixture to the desired reaction temperature.

While it is contemplated that a wide variety of reaction temperatures and pressures may be used, depending on relevant factors such as the catalyst being used and the most desired reaction product, it is generally preferred that at least a portion of the addition step is carried out at a reaction temperature of from 5°C to 1000°C, more preferably 5°C to 500°C, more preferably 5°C to 200°C and even more preferably from 40°C to 60°C for reactors which are preferably maintained at a pressure of from 108 to 10443 kPa (1 to 1500 psig) and even more preferably from 239 to 377 kPa (20 to 40 psig).

In certain preferred embodiments, the compound of formula (III) and the compound of formula (IV) are introduced into an appropriate reaction vessel in the form of a gas and the reactor is preferably maintained at a temperature of about 50°C and the reactor is preferably maintained at a pressure of 308 kPa (30 psig).

In preferred embodiments the conversion of the formula (III) compound, is preferably at least about 15%, more preferably at least about 20%, and selectivity to compounds of formula I is preferably at least about 50%, more preferably at least about 70%, and even more preferably at least about 75%.

### II. FORMATION OF THE COMPOUND OF FORMULA II

The methods of the present invention comprise converting a compound of formula (I) to a fluorolefin
of formula (II).

In certain preferred embodiments, the present converting step is carried out under conditions effective to provide a formula (I) conversion of at least about 40%, more preferably at least about 55%, and even more preferably at least about 70%. In certain preferred embodiments the conversion is at least about 90%, and more preferably about 100%. Further in certain preferred embodiments, the conversion of the compound of formula I to produce a compound of formula II is conducted under conditions effective to provide a formula II selectivity of at least about 25%, more preferably at least about 40%, more preferably at least about 70%, and even more preferably at least about 90%.

This reaction step can be carried out in the liquid phase or in the gas phase, or in a combination of gas and liquid phases, and it is contemplated that the reaction can be carried out batch wise, continuous, or a combination of these.

### A. GAS PHASE DEHYDROHALOGENATION

One preferred reaction step in accordance may be described by those reactions in which the compound of formula (I) comprises a compound
of formula (IA)
which is 1,1,1,2,2-pentafluoro-3chloropropane (CF₃C F₂CH₂Cl). By way of illustration, an embodiment involving this compound may be shown to proceed by the following reaction, where the reducing agent is hydrogen:

**CF₃CF₂CH₂Cl + H₂ → CF₃CF=CH₂ + HCl + HF**

Although applicant does not intend to be bound to or limited by any particular theory of operation, is believed that in some embodiments the above noted reaction proceeds by the formation of another compound, namely, CF₃C F₂CH₃, which is generated as an intermediate or byproduct of the reaction with hydrogen, methane or other dehydrogenating agent. In accordance with such theory, the intermediate formula (IA) compound is then converted to the desired compound of formula (II), namely HFO-1234yf, under the existing reaction conditions, and preferably on the surface of the catalyst.

In an alternative to the above reaction, the reducing agent comprises methane and the reaction is represented, without limitation, as follows:

**CF₃CF₂CH₂Cl** + **CH₄ → CF₃CF=CH₂** + **CH₃Cl** + **HF**

In certain preferred embodiments, the stream containing the compound of formula (I), and preferably (IA) is preheated, primarily to avoid condensation, to a temperature of from 50°C to 90°C, preferably 60°C to 70°C, and introduced into a reaction vessel. The appropriate amount of the reducing agent, which is preferably from 0.1 % to 500% of the stoichiometric amount, is then added to the reaction vessel. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to the desired reaction temperature.

Thus, it is contemplated that the dehydrohalogenation reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a carbon- and/or metal-based catalyst, such as activated carbon, palladium on carbon, palladium-based catalyst (including palladium on carbon and palladium on aluminum oxides), and ruthenium-based catalysts (including ruthenium on aluminum oxides). It is expected that many other catalysts may be used depending on the requirements of particular embodiments in view of the teachings contained herein. Of course, two or more any of these catalysts, or other catalysts not named here, may be used in combination.

In general it is preferred that the catalysts are fluorinated, preferably for a period of from about several hours (eg, 6 hours). In preferred embodiments, fluorination of the catalysts comprises exposing the catalyst to a stream of HF at about reaction temperature and under slight pressure, for example 34 to 1034 kPa (5-150 psia).

The gas phase dehydrohalogenation reaction may be conducted, for example, by introducing a gaseous form of a compound of formula (I), and preferably (1A) and a gaseous form of the reducing agent (and/or dehydrohalogenation agent), into a suitable reaction vessel or reactor. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable reduction/dehydrohalogenation catalyst, with suitable means to heat the reaction mixture to the desired reaction temperature.

While it is contemplated that a wide variety of reaction temperatures may be used, depending on relevant factors such as the catalyst being used and the most desired reaction product, it is generally preferred that the reaction temperature for the dehydrohalogentation step, particularly where the formula (I) compound comprises (and even more preferably consists essentially of compounds of formula (1A)) is from 400°C to 800°C, preferably

400°C to 700°C. For such formula (1A) embodiments in which the reducing agent comprises, and even more preferably consists essentially of hydrogen, the reaction temperature for the dehydrohalogentation step is preferably from 450°C to 600°C, more preferably from 450°C to 550°C. For such formula (1A) embodiments in which the reducing agent comprises, and even more preferably consists essentially of methane, the reaction temperature for the dehydrohalogentation step is preferably from 500°C to 700°C, more preferably from 600°C to 700°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum, and in certain preferred embodiments is from 103 to 827 kPa (15 to 120 psia).

In certain embodiments, an inert diluent gas, such as nitrogen, may be used in combination with the other reactor feeds. When such a diluent is used, it is generally preferred that the compound of formula (I) comprise from about 5% to greater than 99% by weight based on the combined weight of diluent and formula (I) compound.

It is contemplated that the amount of catalyst use will vary depending on the particular parameters present in each embodiment. In certain preferred embodiments, the contact time is from 0.1 seconds to 1000 second, and preferably from 2 seconds to 50 seconds. For embodiments in which the compound of formula (I) comprises or consists essentially of a compound of formula (IA) and the reducing agent comprises or consists essentially of hydrogen, and where the desired product of formula (II) is HFO-1234yf, applicants have found that it is preferred to use as the catalyst a carbon-based catalyst, such as activated carbon, or palladium-based catalyst, or a catalyst comprising palladium and carbon, such as a palladium on carbon catalyst.

For embodiments in which the compound of formula (I) comprises or consists essentially of a compound of formula (IA) and the reducing agent comprises or consists essentially of methane, and where the desired product of formula (II) is HFO-1234yf, applicants have found that it is preferred to use as the catalyst a carbon-based catalyst, such as activated carbon, or a catalyst based on a Period 6 metal (particularly Cs and Ba) such as BaNO₃ or CsNO₃ (including in combination with aluminum-based catalyst or catalyst support, such as Al₂O₃), or Ni-based catalyst (such as Ni mesh) and combinations of these.

Preferably in such dehydrofluorination embodiments as described in this section, the conversion of the formula (I) compound is at least about 50%, more preferably at least about 65%, and even more preferably at least about 90%. Preferably, the selectivity to HFO-1234yf is at least about 70%, more preferably at least about 80% and more preferably at least about 90%.

### B. LIQUID PHASE REDUCTION

One preferred reaction step in accordance may be described by those reactions in which the compound of formula (I) comprises a compound
formula (IB)
which is 3,3,3,2-tetrafluoro-1-chloro-1-propene (CF₃CF=CHCl).

It is contemplated that the reduction reaction step may be performed using a wide variety of process parameters and process conditions in view of the overall teachings contained herein. However, it is preferred in certain embodiments that this reaction step comprise a liquid phase reaction, preferably in the presence of catalyst, and even more preferably in the presence of a catalyst contained in a liquid carrier, such as a solvent for at least one or more of the organic reactants.

Although it is contemplated that many solvents and catalysts will be adaptable for use in connection with these preferred embodiments, it is generally preferred that the solvent comprises tetrahydrofuran, dioxane and the like, and any combinations of solvents including these. In such preferred embodiments, the catalysts preferably include palladium-based catalyst. In certain preferred embodiments the palladium based catalyst comprises, and in certain embodiments consists essentially of tetrakis(triphenylphosphine)palladium(0), [ Pd(PPh₃)₄ and/or tris(dibenzlideneacetone)dipalladium(0), Pd₂(dba)₃ and combinations of these. In certain preferred embodiments, ligands for the catalyst are included in the reaction mixture, and although many ligands are believed to adaptable for use with the preferred catalyst systems of the present invention, in certain embodiments the ligands comprise tetraributyl phosphine, ammonium formate and combinations of these and/or other ligands. It is expected that other catalysts may be used depending on the requirements of particular embodiments in view of the teachings contained herein. Of course, two or more any of these catalysts, or other catalysts not named here, may be used in combination.

The liquid phase reduction reaction may be conducted, for example, by introducing the solvent and catalyst into a suitable reaction vessel or reactor. Preferably the vessel is comprised of materials which are resistant to corrosion as Hastelloy, Inconel, Monel and/or fluoropolymers linings.

Prior to its introduction to the reactor, the compound of formula (I), including a compound of formula (IB), is preferably cooled to below its boiling point, and preferably to a temperature of from -5°C to 20°C and introduced into the solvent. The reaction mixture is then preferably brought to a temperature of from -20°C to -50°C, and even more preferably to -30°C to -40°C and then a partial vacuum is applied to pull residual air or O₂ from the reactor. The reaction mixture is then preferably heated, preferably with agitation (such as stirring) to a temperature of from about 10°C to 200°C, more preferably from 20°C to 150°C, and the reaction mixture is preferably maintained at this temperature for a time period of from about 1 hour to about 48 hours, more preferably from about 15 hours to about 30 hours. During this time period the pressure in the reactor may increase in certain embodiments to 1136 kPa (150 psig) to 1825 kPa (250 psig) and even more preferably from 1136 kPa (150 psig) to 1480 kPa (200 psig).

It is contemplated that the amount of catalyst use will vary depending on the particular parameters present in each embodiment. In certain preferred embodiments, the weight ratio of the compound of formula (I) to catalyst is from about 50,000:1 to about 1:1, and even more preferably from about 100:1 to about 1:1.

Preferably in such reduction embodiments as described in this section, the conversion of the formula (IB) compound is at least about 85%, more preferably at least about 95%, and even more preferably about 100%. Preferably, the selectivity to HFO-1234yf is at least about 20%, more preferably at least about 30% and more preferably at least about 40%.

### EXAMPLES

Additional features of the present invention are provided in the following examples.

### Examples 1-11

These examples illustrate gas phase dehydrohalogenation of CF₃CF₂CH₂Cl (HFC-235cb) to CF3CF=CH2 (1234yf). A 56cm (22-inch (1.27cm; ½ inch diameter) Monel tube reactor is charged with 120 cc of catalyst, as specified in Table 1 below. The reactor is mounted inside a heater with three zones (top, middle and bottom). The inlet of the reactor is connected to a pre-heater, which was kept at 300°C by electrical heating. Organic (235cb) is fed from a cylinder kept at 65°C. A flow of reducing agent comprising hydrogen gas and the inert N₂ gas is maintained at a rate as indicated in Table 1 below. The reactor temperature is brought to the temperature indicated in the table. The HFC-235cb is passed through gas-flow controllers into a preheater maintained a temperature of about 300°C. The gas stream coming out of the preheater is passed through the catalyst bed at the desired temperature over a specified period of time and at a pressure of from 119-138 kPa (2.5-5.3 psig). An on-line GC and a GCMS are used to analyze samples taken at the reactor exit line at regular time intervals. Finally, the reactor effluent is introduced into a 20 - 60 % KOH scrubber solution, and the effluent from the scrubber solution is then condensed to collect the products. The desired product CF₃CF=CH₂ (1234yf) is then isolated from the mixture by distillation. The conversion of HFC-235cb is from 50% to 100% and the selectivity to HFO-1234yf is from 60% to 100%, depending on the reaction conditions. The major byproducts were CF₃CF₂CH₃ (HFC-245cb), CF₃CF₂Cl (CFC-115), CF₃Cl (CFC-13), and CF₃CF=CHCl.

The results are shown in Table 1 below.

**Table 1**

| CF₃CF₂CH₂Cl (HFC-235cb) + He → CF₃CF=CH₂ (1234yf) + HCl + HF | | | | | | | | |
|---|---|---|---|---|---|---|---|---|
| **Example#** / **Catalyst** | **T, °C** | **H2**/**N2**, **Sccm** | **HFC-235cb, sccm** | **% Conv. of 235cb** | **% Selec. to 1234yf** | **% Selec. to 245cb** | **% Selec. to R^{*}** | **% Selec. to R^{**}** |
| Example 1/ A | 450 | 58/0 | 52 | 76 | 60 | 31 | 4 | 4 |
| Example 2/ A | 500 | 61/0 | 54 | 100 | 90 | 9 | 0 | 1 |
| Example 3/ A | 550 | 61/0 | 54 | 100 | 84 | 6 | 1 | 6 |
| Example 4/ A | 600 | 63/0 | 57 | 97 | 63 | 12 | 0 | 24 |
| Example 5/ B | 520 | 41/100 | 96 | 80 | 60 | 20 | 5 | 10 |
| Example 6/ B | 550 | 41/100 | 138 | 58 | 36 | 40 | 7 | 16 |
| Example 7/ C | 500 | 61/0 | 54 | 87 | 69 | 20 | 4 | 6 |
| Example 8/ D | 500 | 61/0 | 54 | 80 | 63 | 26 | 3 | 7 |
| Example 9/ E | 500 | 61/0 | 63 | 100 | 73 | 16 | 2 | 8 |
| Example 10/ F | 500 | 61/0 | 76 | 91 | 30 | 27 | 10 | 30 |
| Example 11/ G | 500 | 61/0 | 100 | 80 | 28 | 29 | 8 | 32 |

| | | | | | | | | |
|---|---|---|---|---|---|---|---|---|
| Catalysts (100 cc): A is Calgon activated carbon; B is Shiro-Saga activated carbon; C is Aldrich activated carbon; D is NORIT RFC 3 activated carbon; E is 0.5 wl% Pd/C; F is 0.5 wit% Pd/Al203; G is 0.5wt% Ru/Al203 Byproducts: R* is CF₃CF=CHCl and R** is combination of CF3C1 and CF₃CF₂Cl | | | | | | | | |

### Examples 12 - 15

These examples illustrate gas phase hydrodehydrochlorination of CF₃CF2CH₃Cl to CF₃CF=CH₂ (HFO-1234yf) using methane as the reducing agent. The protocol of Examples 1-11 is repeated except as indicated in Table 2 below.

**Table 2**

| CF₃CF₂CH₂Cl (235cb) + CH₄ → CF₃CF=CH₂ (1234yf) + CH₃Cl + HF | | | | | | |
|---|---|---|---|---|---|---|
| **Example#** / **Catalyst** | **T, °C** | **P, kPa (psig)** | **CH₄, sccm** | **HFC-235cb, sccm** | **% Conv. of 235cb** | **% Selec. to 1234yf** |
| Example 12/ H | 560 | 170 (10) | 50 | 50 | 17 | 0 |
| Example 13/ I | 650 | 156 (8) | 100 | 86 | 46 | 26 |
| Example 14/ J | 650 | 150 (7) | 97 | 83 | 52 | 31 |

| **Example#** / **Catalyst** | **T, °C** | **P, kPa (psig)** | **CH₄, sccm** | **HFC-235cb, sccm** | **% Conv. of 235cb** | **% Selec. to 1234yf** |
|---|---|---|---|---|---|---|
| Example 14*/ K | 650 and 500 * | 156 (8) | 96 | 87 | 54 | 40 |
| Example 15/ L | 585 | 122 (3) | 79 | 100 | 77 | 33 |

| | | | | | | |
|---|---|---|---|---|---|---|
| Catalysts (100 cc): H is activated carbon; 13wt% BaNO3/Al203; J is 3wt% BaNO3/Al203 with 0.5wt% CsNO3 used as a promoter; K is a two zone reaction with two catalysts, specifically 50 cc CsNO3 promoted with BaNO3/Al203 is used at 650°C in the first zone and 50 cc Calgon activated carbon is used at 500°C in the second zone; L is Ni mesh. * two zone reaction | | | | | | |

### Example 16

This example illustrates the liquid phase reduction of CF₃CF=CHCl to CF₃CF=CH₂ (1234yf) with Pd(PPh₃)₄ catalyst. A 250 mL Parr reactor/autoclave is charged with 30 mL tetrahydrofuran to which 0.5 grams (0.04 mmol) [tetrakis(triphenylphosphine)palladium(0), [Pd(PPh₃)₄], 10.0 grams of ammonium formate (158 mmol), and 11.4 grams cold (0-10 °C) CF3CF=CHC1(77 mmol) are added under nitrogen. The reactor is sealed immediately, cooled to -30 to -40 °C, and partially evacuated. The contents in the Parr reactor are brought to room temperature and gradually heated to 100°C with stirring. The reactants are maintained at this temperature for 24 hours. During this time, the pressure in the reactor is increased to approximately 1342-1840 kPa (180-200 psig). The reactor is then cooled to 25°C and the volatile materials are collected in an evacuated metal cylinder.

Gas chromatographic (GC) analysis of the volatile materials indicated CF₃CF=CH₂ as the main product with trace amounts of CF₃CF=CHCl and carbon dioxide. The reaction mixture is cooled to 0°C and filtrate is analyzed by gas chromatograph (GC) which indicates approximately a 40% conversion of CF₃CF=CHCl to CF3CF=CH₂. Identity of CF₃CF=CH₂ is confirmed by comparison with a known sample. Purification of the product is accomplished by passing the product through a cold trap to remove CO₂ and unreacted starting material at about -70 °C and then distillation.

### Example 17

This example illustrates the liquid phase reduction of CF₃CF=CHCl to CF₃CF=CH₂ (HFO-1234yf) with Pd₂(dba)₃ catalyst. A 250 mL Parr reactor/autoclave was charged with 30 mL tetrahydrofuran (30 mL) to which 0.456 grams of tris(dibenzlideneacetone)dipalladium(0), Pd₂(dba)₃ (2.0 mmol), 0.8 grains of tributyl phosphine (2.0 mmol), 8.0 grams of ammonium formate (126 mmol), and 11.4 grams of cold (5-10 °C) CF₃CF=CHCl (77 mmol) are added under nitrogen. The reactor is sealed immediately, cooled to the range of from about -30°C to about -40 °C, and partially evacuated. The contents in the Parr reactor are brought to room temperature and then gradually heated to 100°C with stirring. The reactants are maintained at this temperature for 24 hours during which the internal pressure rises to approximately 1840 kPa (200 psig). The reactor is then cooled to 25°C and the volatile materials are passed through a trap at
-70°C to -78°C and collected in a cold evacuated metal cylinder. Gas chromatographic (GC) analysis of the volatile materials collected indicate CF₃CF=CH₂ as the main product with trace amounts of CF₃CF=CHCl and carbon dioxide. The reaction mixture was cooled to 0 °C and filtrate was analyzed by GC which indicated approximately a 35% conversion of CF₃CF=CHCl to CF₃CF=CH₂.

### Example 18

This example illustrates the liquid phase reduction of CF₃CF=CHCl to CF₃CF=CH₂ (HFO-1234yf) with Pd₂(dba)₃ catalyst. Example 17 is repeated except that dioxane is substituted for tetrahydrafuran as the solvent. The result is essentially the same as Example 17.

### Example 19

This example illustrates the liquid phase reduction of CF₃CF=CHCl to CF₃CF=CH2 (HFO-1234yf) using Pd₂(dba)₃•CHCl₃ catalyst. The reaction is carried out as in Example 17 except that an equivalent amount of catalyst Pd₂(dba)₃ is substituted by tris(dibenzlideneacetone)dipalladium (0) chloroform complex, Pd₂(dba)₃ •CHCl₃. The extent of conversion of CF₃CF=CHCl to CF₃CF=CH₂ was essentially the same as in Example 17.

### Example 20

This example illustrates the liquid phase reduction of CF₃CF=CHCl to CF₃CF=CH₂ (HFO-1234yf) with Pd(PPh₃)₄ catalyst in tetrahydrofuran. A 250 mL Parr Reactor/autoclave was charged with 30 mL of tetrahydrofuran to which 0.5 grams of tetrakis(triphenylphosphine)palladium(0), Pd(PPh₃)₄ (0.04 mmol), 10 grams of ammonium formate (158 mmol), and 11.4 grams of CF3CF=CHCl (61 mmol) were added under nitrogen. The reactor is sealed immediately, cooled to -30 to -40 °C, and partially evacuated. The contents in the Parr reactor were brought to room temperature and gradually heated to 100°C with constant stirring. The reactants are maintained at this temperature for 24 hours. The reactor was then cooled to 25°C and the volatile materials were collected in an evacuated metal cylinder. Gas chromatographic (GC) analysis of the volatile materials indicated that CF₃CF=CH₂ and CF₃CH=CHCI are present at a ratio of about 57:42. Carbon dioxide was also present. The reaction mixture was cooled to 0°C and filtered under pressure, the filtrate was analyzed by GC which indicated about a 40% conversion of CF₃CF=CHCl to CF₃CH=CH₂.

### Examples 21 - 28 (Reference Example)

These examples illustrate gas phase dehydrohalogenation of CF₃CF₂CH₃ (HFC-245cb) to CF₃CF=CH₂ (HFO-1234yf). A 56cm (22-inch (1.27cm; ½ inch diameter) Monel tube reactor is charged with 120 cc of catalyst, as specified in Table I below. The reactor is mounted inside a heater with three zones (top, middle and bottom). The inlet of the reactor is connected to a pre-heater, which was kept at 300°C by electrical heating. Organic (HFC-245cb) is fed from a cylinder kept at 65°C. A flow of reducing agent comprising hydrogen gas is maintained at a rate as indicated in Table 3 below. The reactor temperature is brought to the temperature indicated in the table. The HFC-245cb is passed through gas-flow controllers into a preheater maintained a temperature of about 300°C. The gas stream coming out of the preheater is passed through the catalyst bed at the desired temperature over a specified period of time and at a pressure of from 119-138 kPa (2.5-5.3 psig). An on-line GC and a GCMS are used to analyse samples taken at the reactor exit line at regular time intervals. Finally, the reactor effluent is introduced into a 20 - 60 % KOH scrubber solution, and the effluent from the scrubber solution is then condensed to collect the products. The desired product CF₃CF=CH₂ (HFO-1234yf) is then isolated from the mixture by distillation. The conversion of HFC-245cb is from 30% to 70% and the selectivity to HFO-1234yf is from 90% to 100%, depending on the reaction conditions.

The results are shown in Table 3 below.

**Table 3**

| CF₃CF₂CH₃ (BFC-245cb) → CF₃CF=CH₂ (1234yf) | | | | | |
|---|---|---|---|---|---|
| **Example**# / **Catalyst** | **T, °C** | **H2/N2, sccm** | **HFC-245cb, sccm** | **% Conv. of 245cb** | **% Selec. to 1234yf** |
| Example 21/ M | 575 | 0 | 65 | 79 | 63 |
| Example 22/ N | 575 | 0 | *68* | 82 | 57 |
| Example 23/ O | 575 | 0 | 73 | 73 | 61 |
| Example 24/ P | 575 | 0 | 68 | 84 | 59 |
| Example 25/ P | 575 | 20 | 68 | 89 | 73 |
| Example 26/ Q | 550 | 0 | 69 | 92 | 53 |
| Example 27/ R | 550 | 0 | 67 | 93 | 33 |
| Example 28/ S | 550 | 0 | 69 | 73 | 46 |

| | | | | | |
|---|---|---|---|---|---|
| Catalysts (100 cc): M is NORIT RFC 3; N is Shiro-Saga activated carbon; O is Aldrich activated carbon; P is Calgon activated carbon; Q is 0.5 wt% Pd/C; R is 0.5 wt% PVC; S is Ni-mesh | | | | | |

### Example 29

This example illustrates the addition formation of compounds of formula (I) by the reaction of TFE with CH₂FCl in a gas phase reaction. Into a 1.27cm (1/2 inch) flow reactor (Monel) 50 grams of freshly prepared catalyst (as indicated below) are charged. CF₂=CF₂ (TFE) and CH₂FCl (R31) are passed through a mass flow controller with a desired flow rate (as indicated below) to the preheater from respective cylinders connected with regulators. The preheater was connected to the reactor and always kept 10°C below the reactor temperature. The reactor was uniformly heated to the desired temperature by an external heating element with an automatic control. The exit line from the reactor was connected to an on-line GC and GCMS for analysis. A 15 wt% KOH scrubber solution was used at 50°C to neutralize acids coming out from the reactor. The gas stream coming out of the scrubber solution was then condensed in a cylinder under liquid N2 and then finally fractionated (distilled) to isolate products. SbF₅/C and AlCl₃/C are used as the catalyst. At 50°C and under 206kPa (30 psig) reactor pressure, when 50 sccm of TFE and 150 sccm of R31 were passed over SbF5/C to achieve a 26% conversion of TFE and an 82% selectivity to CF₃CF₂CH₂Cl. When AlCl3/C is used as the catalyst, a 35% conversion and 78% selectivity to CF₃CF₂CH₂Cl was obtained.

### Example 30

This example illustrates the addition formation of compounds of formula (I) by the reaction of CTFE with CH₂FCl in a gas phase reaction. Example 29 is repeated except that CTFE is used in place of TFE. The major reaction products include CF₃CClFCH₂Cl and F₂ClCCF₂CH₂Cl at a 21 % conversion (CTFE) level.

### Example 31

This example illustrates the formation of compounds of formula (I) by the reaction of TFE with CH₂FCl in a gas phase reaction. Into a 300 ml autoclave, 0.1 mol C₂F₄ was reacted with 0.2 mol CH₂ClF in the presence of 0.05 mol of AlCl₃ at 20-30° for 3 hr to give 60% yield to CF₃CF₂CH₂Cl which was then isolated and purified by distillation.

### Example 32

This example illustrates the formation of compounds of formula (I) by the reaction of TFE with CH₂FCl in a gas phase reaction. Into a 300 ml autoclave, 0.1 mol C₂F₄ was reacted with 0.2 mol CH₂ClF in the presence of 0.05 mol of AICl₃ at 20-30° for 3 hr to give 60% yield to CF₃CF₂CH₂Cl which was then isolated and purified by distillation.

### Example 33

This example illustrates the formation of compounds of formula (I) by the reaction of CTFE with CH₂FCl in a gas phase reaction. Into a 300 ml autoclave, 0.1 mol C F₂=CFCl was reacted with 0.2 mol CH₂CIF in the presence of 0.05 mol of AICl₃ at 20-30° for 3 hr to give 60% yield to CF₃CClFCH₂Cl and F₂ClCF₂CH₂Cl which was then isolated and purified by distillation.

## Claims

1. A method for the preparation of 2,3,3,3-tetrafluoro-1-propene (CF₃CF=CH₂: HFO-1234yf) comprising: a) adding chlorofluoromethane (CH₂FCl) to a compound of formula CF₂=CY₂ selected from tetrafluoroethylene (CF₂=CF₂) or CTFE (CF₂=CFCl): and b) converting the obtained 1-chloro-2,2,3,3,3-pentafluoropropane (CF₃CF₂CH₂Cl; HCFC-235cb) or 1-chloro-2,3,3,3-tetrafluoro-1-propene (CF₃CF=CHCl).

2. A method as claimed in claim 1 wherein said step b) comprises converting the obtained 1-chloro-2,2,3,3,3-pentafluoropropane (CF₃CF₂CH₂Cl) by a dehydrohalogenation reaction or 1-chloro-2,3,3,3-tetrafluoropropene (CF₃CF=CHCl) by a reduction reaction.

3. A method according to claim 2, wherein the dehydrohalogenation is carried out in the gas phase.

4. A method as claimed in claim 3, wherein the dehydrohalogenation is carried out in the
presence of a catalyst.

5. A method according to claim 3 or claim 4, wherein the catalyst is selected from carbon- and/or metal based catalysts, palladium-based catalysts, ruthenium-based catalysts and combinations thereof.

6. A method according to claim 5, wherein the catalyst is selected from activated carbon, palladium on carbon, palladium on aluminium oxides, ruthenium on aluminium oxides and combinations thereof.

7. A method according to claim 2 wherein the reduction is carried out in the liquid phase.

8. A method according to claim 7, wherein the reduction is carried out in the presence of a catalyst.

9. A method according to claim 7 or claim 8, wherein the catalyst is a palladium-based catalyst.

10. A method according to any preceding claim, wherein step a) is carried out in the gas phase.

11. A method according to claim 10 wherein step a) is carried out in the presence of a catalyst.

12. A method according to claim 10 or claim 11, wherein the catalyst is supported or unsupported and is a metal-based catalyst.

13. A method according to claim 12 wherein the metal-based catalyst is antimony-based catalyst, aluminium-based catalysts and iron-based catalysts.

## Patentansprüche

1. Verfahren zur Herstellung von 2,3,3,3-Tetrafluor-1-propen (CF₃CF=CH₂; HFO-1234yf), das umfasst:
a) Zugeben von Chlorfluormethan (CH₂FCl) zu einer Verbindung der Formel CF₂=CY₂, die ausgewählt ist aus Tetrafluorethylen (CF₂=CF₂) oder CTFE (CF₂=CFCl); und
b) Umwandeln des erhaltenen 1-Chlor-2,2,3,3,3-Pentafluorpropans (CF₃CF₂CH₂Cl; HCFC-235cb) oder 1-Chlor-2,3,3,3-Tetrafluor-1-Propens (CF₃CF=CHCl).

2. Verfahren nach Anspruch 1, wobei der Schritt b) das Umwandeln des erhaltenen 1-Chlor-2,2,3,3,3-Pentafluorpropans (CF₃CF₂CH₂Cl) durch eine Dehydrohalogenierungsreaktion oder 1-Chlor-2,3,3,3-Tetrafluorpropens (CF₃CF=CHCl) durch eine Reduktionsreaktion umfasst.

3. Verfahren nach Anspruch 2, wobei die Dehydrohalogenierung in der Gasphase durchgeführt wird.

4. Verfahren nach Anspruch 3, wobei die Dehydrohalogenierung in der Gegenwart eines Katalysators durchgeführt wird.

5. Verfahren nach Anspruch 3 oder 4, wobei der Katalysator ausgewählt ist aus Kohlenstoff- und/oder Metall-basierten Katalysatoren, Palladium-basierten Katalysatoren, Ruthenium-basierten Katalysatoren und Kombinationen davon.

6. Verfahren nach Anspruch 5, wobei der Katalysator ausgewählt ist aus Aktivkohle, Palladium auf Kohlenstoff, Palladium auf Aluminiumoxiden, Ruthenium auf Aluminiumoxiden und Kombinationen davon.

7. Verfahren nach Anspruch 2, wobei die Reduktion in der Flüssigphase durchgeführt wird.

8. Verfahren nach Anspruch 7, wobei die Reduktion in der Gegenwart eines Katalysators durchgeführt wird.

9. Verfahren nach Anspruch 7 oder 8, wobei der Katalysator ein Palladium-basierter Katalysator ist.

10. Verfahren nach einem der vorhergehenden Ansprüche, wobei Schritt a) in der Gasphase durchgeführt wird.

11. Verfahren nach Anspruch 10, wobei Schritt a) in der Gegenwart eines Katalysators durchgeführt wird.

12. Verfahren nach Anspruch 10 oder 11, wobei der Katalysator geträgert oder ungeträgert ist und ein Metall-basierter Katalysator ist.

13. Verfahren nach Anspruch 12, wobei der Metallbasierte Katalysator ein Antimon-basierter Katalysator, Aluminium-basierte Katalysatoren und Eisen-basierte Katalysatoren ist.

## Revendications

1. Procédé pour la préparation de 2,3,3,3-tétrafluoro-1-propène (CF₃CF=CH₂ : HFO-1234yf) comprenant : a) l'addition de chlorofluorométhane (CH₂FCl) à un composé de formule CF₂=CY₂ choisi parmi le tétrafluoroéthylène (CF₂=CF₂) et le CTFE (CF₂=CFCl) ; et b) la conversion du 1-chloro-2,2,3,3,3-pentafluoropropane (CF₃CF₂CH₂Cl ; HCFC-235cb) ou du 1-chloro-2,3,3,3-tétrafluoro-1-propène (CF₃CF=CHCl) obtenu.

2. Procédé selon la revendication 1, dans lequel ladite étape b) comprend la conversion du 1-chloro-2,2,3,3,3-pentafluoropropane (CF₃CF₂CH₂Cl) obtenu par une réaction de déshydrohalogénation ou du 1-chloro-2,3,3,3-tétrafluoroproprène (CF₃CF=CHCl) obtenu par une réaction de réduction.

3. Procédé selon la revendication 2, dans lequel la déshydrohalogénation est effectuée en phase gazeuse.

4. Procédé selon la revendication 3, dans lequel la déshydrohalogénation est effectuée en présence d'un catalyseur.

5. Procédé selon la revendication 3 ou la revendication 4, dans lequel le catalyseur est choisi parmi les catalyseurs à base de carbone et/ou de métal, les catalyseurs à base de palladium, les catalyseurs à base de ruthénium et leurs combinaisons.

6. Procédé selon la revendication 5, dans lequel le catalyseur est choisi parmi le charbon activé, le charbon palladié, les oxydes d'aluminium palladiés, les oxydes d'aluminium ruthéniés et leurs combinaisons.

7. Procédé selon la revendication 2, dans lequel la réduction est effectuée en phase liquide.

8. Procédé selon la revendication 7, dans lequel la réduction est effectuée en présence d'un catalyseur.

9. Procédé selon la revendication 7 ou la revendication 8, dans lequel le catalyseur est un catalyseur à base de palladium.

10. Procédé selon l'une quelconque des revendications précédentes, dans lequel l'étape a) est effectuée en phase gazeuse.

11. Procédé selon la revendication 10, dans lequel l'étape a) est effectuée en présence d'un catalyseur.

12. Procédé selon la revendication 10 ou la revendication 11, dans lequel le catalyseur est supporté ou non supporté et est un catalyseur à base de métal.

13. Procédé selon la revendication 12, dans lequel le catalyseur à base de métal est un catalyseur à base d'antimoine, un catalyseur à base d'aluminium ou un catalyseur à base de fer.
